(19) Europäisches Patentamt
European Patent Office
Office européen des brevets

(11) Veröffentlichungsnummer: **0 332 930 A2**

# (12) EUROPÄISCHE PATENTANMELDUNG

(21) Anmeldenummer: **89103546.1**

(51) Int. Cl.[4]: **C07D 215/56**

(22) Anmeldetag: **01.03.89**

(30) Priorität: **11.03.88 DE 3808118**

(43) Veröffentlichungstag der Anmeldung: **20.09.89 Patentblatt 89/38**

(84) Benannte Vertragsstaaten: **AT BE CH DE ES FR GB GR IT LI NL SE**

(71) Anmelder: **BAYER AG**

**D-5090 Leverkusen 1 Bayerwerk(DE)**

(72) Erfinder: **Schriewer, Michael, Dr.**
**Am Thelen Siefen 1 a**
**D-5068 Odenthal(DE)**
Erfinder: **Krebs, Andreas, Dr.**
**Am Gartenfeld 70**
**D-5068 Odenthal(DE)**
Erfinder: **Petersen, Uwe, Dr.**
**Auf dem Forst 4**
**D-5090 Leverkusen 1(DE)**
Erfinder: **Grohe, Klaus, Dr.**
**Am Wasserturm 10**
**D-5068 Odenthal(DE)**

## (54) Verfahren zur Herstellung von 1-Cyclopropyl-chinoloncarbonsäuren und deren Derivaten.

(57) Die Erfindung betrifft ein Verfahren zur Herstellung von Chinoloncarbonsäurederivaten der Formel (I)

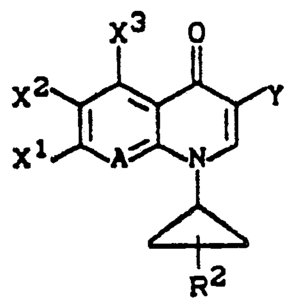

(I)

unter Verwendung neuer Zwischenprodukte der Formel (IV)

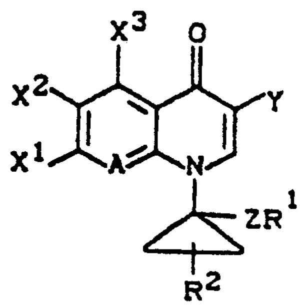

IV,

wobei die Reste $R^2$, $X^1$, $X^2$, $X^3$, A, Y, Z und $R^1$ die in der Beschreibung angegebene Bedeutung haben.

EP 0 332 930 A2

## Verfahren zur Herstellung von 1-Cyclopropyl-chinoloncarbonsäuren und deren Derivaten

Die Erfindung betrifft neue Zwischenprodukte für 1-Cyclopropylchinoloncarbonsäuren und ein Verfahren zur Herstellung von Cyclopropylchinoloncarbonsäuren unter Verwendung dieser Zwischenprodukte.

In 7-Stellung aminsubstituierte 4-Chinolon-3-3-carbonsäuren sind für ihre gute antibakterielle Wirkung bekannt. Aus dieser Gruppe ragen insbesondere die 1-Cyclopropyl-Verbindungen aufgrund ihres hohen Wirkniveaus heraus.

Beispielhaft für dieser Klasse sei die 1-Cyclopropyl-6-fluor-1,4-dihydro-7-(1-piperazinyl)-4-oxo-3-chinolincarbonsäure (Ciprofloxacin) erwähnt.

Das erfindungsgemäße Verfahren erlaubt es nun, die aufgrund der excellenten Wirkung wertvollen 1-Cyclopropyl-chinoloncarbonsäuren bzw. deren Derivate herzustellen. Die Erfindung betrifft ein Verfahren zur Darstellung von Chinoloncarbonsäurederivaten der Formel (I)

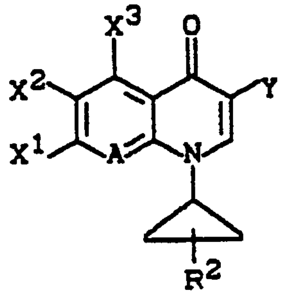

(I)

in der

$R^2$ für Wasserstoff, $C_1$-$C_3$-Alkyl oder Halogen steht,

$X^1$ Wasserstoff, $C_1$-$C_3$-Alkyl, Halogen oder $NR^8R^9$ bedeutet, wobei

$R^8$ und $R^9$ gemeinsam mit dem Stickstoffatom, an das sie gebunden sind, einen 5- oder 6-gliedrigen Ring bilden, der als Ringglieder zusätzlich die Atome bzw. die Gruppen $>NR^{10}$ oder O enthalten kann und der gegebenenfalls ein- bis zweifach durch $C_1$-$C_3$-Alkyl, Hydroxy, Methoxy oder $NR^{11}R^{12}$ substituiert sein kann, wobei

$R^{10}$ für Wasserstoff, $C_1$-$C_3$-Alkyl, Acetyl oder t-Butoxycarbonyl steht,

$R^{11}$ für Wasserstoff oder $C_1$-$C_3$-Alkyl steht,

$R^{12}$ Wasserstoff, $C_1$-$C_3$-Alkyl, Acetyl oder t-Butoxycarbonyl bedeutet,

$X^2$ und $X^3$ gleich oder verschieden sein können und für Wasserstoff, Nitro oder Halogen stehen,

A für Stickstoff oder einen Rest C-$X^4$ steht, wobei

$X^4$ für Wasserstoff, $C_1$-$C_3$-Alkyl, Alkoxy mit 1 bis 3 Kohlenstoffatomen, Alkylmercapto mit 1 bis 3 Kohlenstoffatomen, Halogen, Nitro, Cyano oder Alkoxycarbonyl mit bis zu 3 Kohlenstoffatomen im Alkoholteil steht und

Y eine Nitrilgruppe, eine Estergruppe $COOR^5$ oder eine Säureamidgruppe $CONR^6R^7$ darstellt, wobei $R^5$ für $C_1$-$C_3$-Alkyl steht und $R^6$ und $R^7$ gleich oder verschieden sein können und für Wasserstoff oder $C_1$-$C_3$-Alkyl stehen und $R^6$ gegebenenfalls substituiertes Phenyl sein kann,

dadurch gekennzeichnet, daß man in einem ersten Schritt Chinoloncarbonsäurederivate der Formel (II)

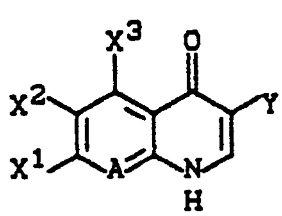

(II)

in der

$X^1$, $X^2$, $X^3$, A und Y die oben angegebene Bedeutung haben mit Cyclopropanderivaten der Formel (III)

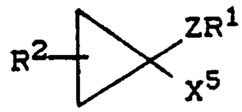

(III)

in der
$X^5$ für Halogen steht,
$R^1$ Alkyl mit 1 bis 3 Kohlenstoffatomen bedeutet,
$R^2$ die oben angegebene Bedeutung hat und
Z Sauerstoff oder Schwefel bedeutet
under $HX^5$-Abspaltung zu 1-Cyclopropylchinoloncarbonsäurederivaten der Formel (IV) nach folgenden Schema

II + III

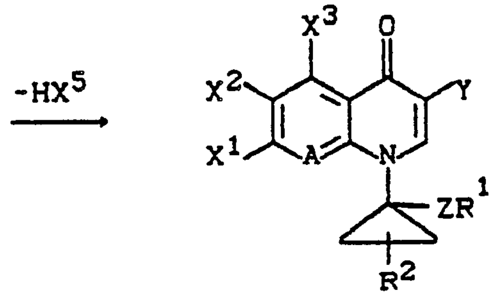


IV,

umsetzt,
die darauf

IV

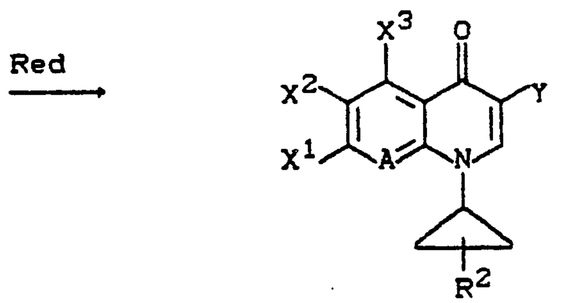


I

durch reduktive Entfernung des Restes $ZR^1$ in die 1-Cyclopropylchinoloncarbonsäurederivate I überführt werden.

Bevorzugt ist das Verfahren zur Darstellung von Chinoloncarbonsäurederivaten der Formel (I)

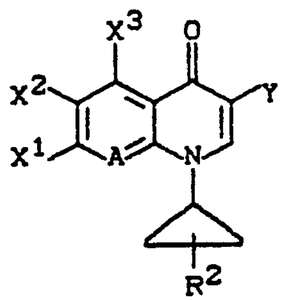

(I)

in der

$R^2$ für Wasserstoff oder Halogen steht,
$X^1$ Halogen oder $NR^8R^9$ bedeutet, wobei
$R^8$ und $R^9$ gemeinsam mit dem Stickstoffatom, an das sie gebunden sind, einen 5- oder 6-gliedrigen Ring bilden, der als Ringglieder zusätzlich die Atome bzw. die Gruppen $>NR^{10}$ oder O enthalten kann und der gegebenenfalls ein- bis zweifach durch $C_1$-$C_3$-Alkyl, Hydroxy, Methoxy oder $NR^{11}R^{12}$ substituiert sein kann, wobei
$R^{10}$ für Wasserstoff, $C_1$-$C_3$-Alkyl, Acetyl oder t-Butoxycarbonyl steht,
$R^{11}$ für Wasserstoff oder $C_1$-$C_3$-Alkyl steht,
$R^{12}$Wasserstoff, $C_1$-$C_3$-Alkyl, Acetyl oder t-Butoxycarbonyl bedeutet,
$X^2$ für Fluor steht,
$X^3$ Wasserstoff, Nitro oder Halogen bedeutet,
A für Stickstoff oder einen Rest C-$X^4$ steht, wobei $X^4$ für Wasserstoff, Halogen oder Nitro steht und
Y eine Nitrilgruppe oder eine Estergruppe $COOR^5$ darstellt, wobei
$R^5$ für $C_1$-$C_3$-Alkyl steht,
dadurch gekennzeichnet, daß man in einem ersten Schritt Chinoloncarbonsäurederivate der Formel (II)

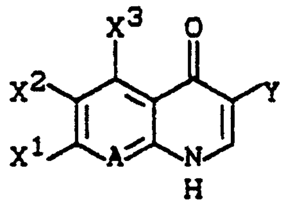

(II)

in der
$X^1$, $X^2$, $X^3$, A und Y die oben angegebene Bedeutung haben mit Cyclopropanderivaten der formel (III)

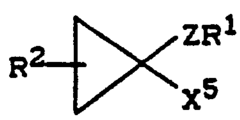

(III)

in der
$X^5$ für Chlor oder Brom steht,
$R^1$ Alkyl mit 1 bis 3 Kohlenstoffatomen bedeutet,
$R^2$ die oben angegebene Bedeutung hat und
Z Sauerstoff oder Schwefel bedeutet under $HX^5$-Abspaltung nach folgendem Schema

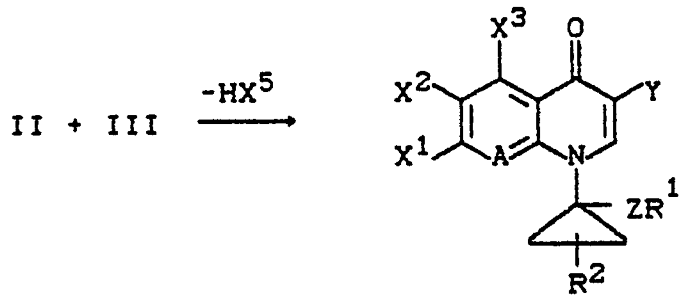


IV,

zu 1-Cyclopropylchinoloncarbonsäurederivaten der Formel (IV) umsetzt, die durch reduktive Entfernung des Restes $ZR^1$.

IV 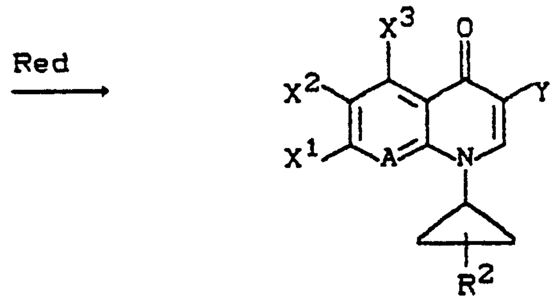

I

in die 1-Cyclopropylchinoloncarbonsäurederivate I überführt werden. Besonders bevorzugt ist das Verfahren zur Darstellung von Chinoloncarbonsäurederivaten der Formel (I)

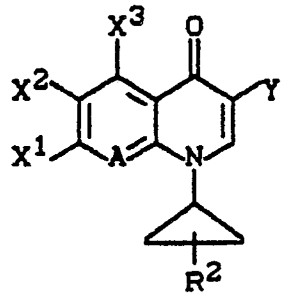
(I)

in der
$R^2$ für Wasserstoff, Fluor oder Chlor steht,
$X^1$ Chlor, Fluor oder $NR^8R^9$ bedeutet, wobei
$R^8$ und $R^9$ gemeinsam mit dem Stickstoffatom, an das sie gebunden sind, einen 5- oder 6-gliedrigen Ring bilden, der als Ringglieder zusätzlich die Gruppe
$>NR^{10}$ enthalten kann und der gegebenenfalls ein- bis zweifach durch Methyl, Hydroxy, Methoxy oder $NR^{11}R^{12}$ substituiert sein kann, wobei
$R^{10}$ für Wasserstoff, Methyl, Acetyl oder t-Butoxycarbonyl steht,
$R^{11}$ für Wasserstoff oder Methyl steht,
$R^{12}$ Wasserstoff, Acetyl oder tert.-Butoxycarbonyl bedeutet,
$X^2$ für Fluor steht,
$X^3$ Waserstoff oder Nitro bedeutet,
A für C-$X^4$ steht, wobei
$X^4$ für Wasserstoff, Methyl, Fluor oder Chlor steht und
Y eine Estergruppe $COOR^5$ darstellt, wobei $R^5$ für Methyl oder Ethyl steht,
dadurch gekennzeichnet, daß man in einem ersten Schritt Chinoloncarbonsäurederivate der Formel (II)

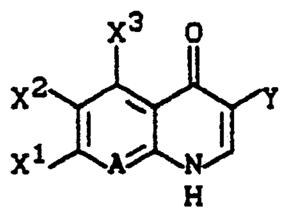
(II)

in der
$X^1$, $X^2$, $X^3$, A und Y die oben angegebene Bedeutung haben mit Cyclopropanderivaten der Formel (III)

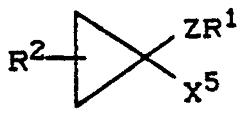

(III)

in der
$X^5$ für Brom steht,
$R^1$ Ethyl bedeutet,
$R^2$ die oben angegebene Bedeutung hat und
Z Sauerstoff oder Schwefel bedeutet unter $HX^5$-Abspaltung nach folgendem Schema

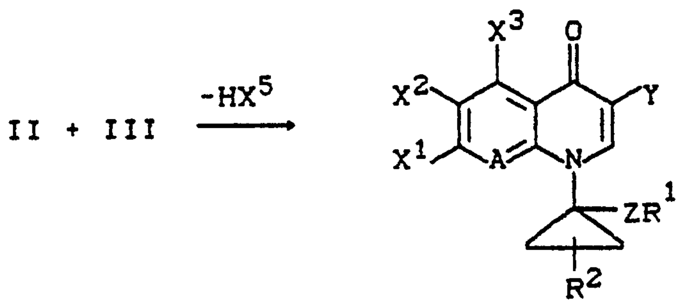


IV,

zu 1-Cyclopropylchinoloncarbonsäurederivaten der Formel (IV) umsetzt, die durch reduktive Entfernung des Restes $ZR^1$

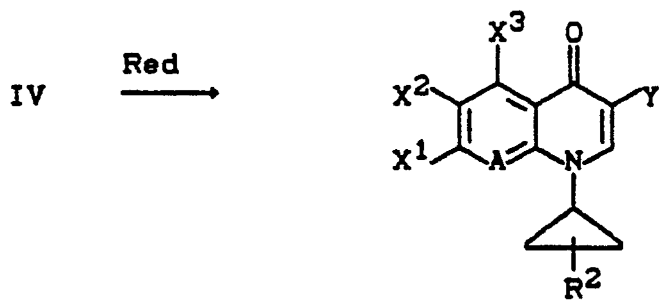


I

in die 1-Cyclopropylchinoloncarbonsäurederivate I überführt werden.

Die Verbindungen der Formel IV sind ebenfalls Gegenstand der Erfindung.

Die Chinoloncarbonsäurederivate I können auf bekannte Weise sauer oder alkalisch in die antibakteriell wirksamen Chinoloncarbonsäuren umgewandelt werden.

Verwendet man beispielsweise 7-Chlor-6-fluor-1,4-dihydro-4-oxo-3-chinoloncarbonsäureethylester und 1-Brom-1-ethoxy-cyclopropan als Ausgangsstoffe, so kann der Reaktionsablauf durch folgendes Formelschema wiedergegeben werden:

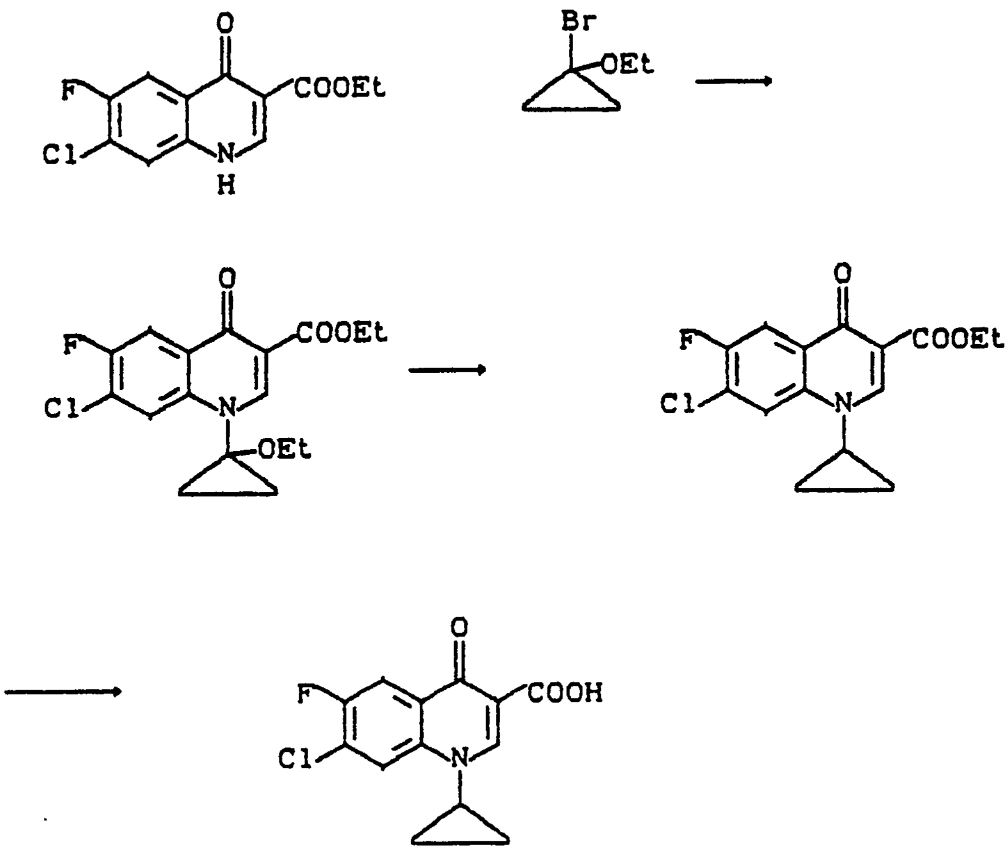

Die als Ausgangsstoffe verwendeten Verbindungen der Formel (II) sind bekannt oder können nach bekannten Methoden hergestellt werden (EP 87106123.0). Als Beispiele seien genannt:
7-Chlor-6-fluor-4-hydroxy-3-chinolincarbonsäuremethylester,
6,7-Difluor-4-hydroxy-3-chinolincarbonsäureethylester,
6,7-Difluor-4-hydroxy-3-chinolincarbonsäuremethylester,
6,7,8-Trifluor-4-hydroxy-3-chinolincarbonsäureethylester,
8-Chlor-6,7-difluor-4-hydroxy-3-chinolincarbonsäureethylester,
7-Chlor-4-hydroxy-8-methyl-3-chinolincarbonsäureethylester,
7-Chlor-6-fluor-4-hydroxy-8-methyl-3-chinolincarbonsäureethylester,
6-Fluor-4-hydroxy-7-methyl-3-chinolincarbonsäureethylester,
6,7-Dichlor-4-hydroxy-3-chinolincarbonsäureethylester.

Die als Ausgangsstoffe verwendeten Cyclopropanderivate der Formel III sind bekannt (J. Org. Chem. 1985, 3255; Recl. Trav. Chim, Pays-Bas 100, 184 (1981).

Als Beispiele seien genannt:
1-Chlor-1-methoxy-cyclopropan, 1-Brom-1-methoxy-cyclopropan, 1-Brom-1-ethoxycyclopropan, 1-Brom-1-methyl-mercapto-cyclopropan.

Die Substitutionsreaktionen II + III → IV werden in einem Temperaturbereich von 20 bis 120 °C, bevorzugt 60 bis 100 °C durchgeführt.

Als Verdünnungsmittel für diese Reaktion können beispielsweise verwendet werden: Dimethylformamid, N-Methylpyrrolidon, Tetramethylharnstoff, Dimethylacetamid.

Für diese Reaktion können als Säurebinder anorganische und organische Basen Verwendung finden. Beispielhaft seien genannt:
1,4-Diazabicyclo[2.2.2]octan, 1,8-Diazabicyclo[5.4.0]-undec-7-en, 1,5-Diazabicyclo[4.3.0]non-5-en, Pyridin, Dimethylbenzylamin, Kaliumtertiärbutylat, Kaliumcarbonat, Natriumhydrogencarbonat, Natriumhydrid.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Als Reduktionsmittel für den Reduktionsschritt IV → I finden im Fall Z = O beispielsweise Verwendung: Natriumcyanoborhydrid, Natriumborhydrid, Lithiumaluminiumhydrid und bevorzugt ein 1:1 Gemisch aus

Natriumborhydrid und Bortrifluorid-Diethylether-Komplex. Im Fall von Z = S finden bekannte Entschwefelungsreagenzien wie z.B. Raney-Nickel Verwendung. Die Reaktion kann z.B. in einem Verdünnungsmittel wie Tetrahydrofuran oder Dioxan durchgeführt werden.

Der Temperaturbereich liegt zwischen 0 und 100 °C, bevorzugt zwischen 0 und 80 °C.

Das folgende Beispiel erläutert die Erfindung.

Beispiel

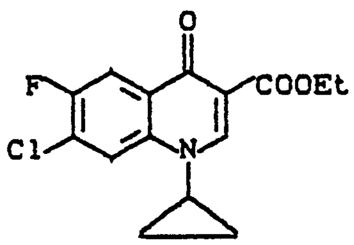

7-Chlor-1-cyclopropyl-6-fluor-1,4-dihydro-4-oxo-3-chinolincarbonsäureethylester

2,7 g (0,01 mol) 7-Chlor-6-fluor-4-hydroxy-3-chinolincarbonsäureethylester, 2,97 g (0,03 mol) Kaliumcarbonat und 4,95 g (0,03 mol) 1-Brom-1-ethoxy-cyclopropan werden in 100 ml N-Methylpyrrolidon 8 Stunden auf 80 °C erhitzt. Anschließend entfernt man alles Flüchtige im Hochvakuum. Der Rückstand wird in Wasser aufgenommen und mit Methylenchlorid extrahiert. Die organische Phase wird über Natriumsulfat getrocknet und eingeengt. Den Rückstand reinigt man durch Chromatographie an Kieselgel mit Methylenchlorid/Methanol 99/1 als Laufmittel.

Zur Reduktion stellt man eine Mischung 0,76 g (0,02 mol) Natriumborhydrid und 2,83 g (0,02 mol) Bortrifluorid-Diethylether-Komplex in 50 ml absolutem Tetrahydrofuran unter Eiskühlung her. Man fügt den 7-Chlor-1-(1-ethoxy-cyclopropyl)-6-fluor-1,4-dihydro-4-oxo-3-chinolincarbonsäureethylester zu und erhitzt 1,5 Stunden unter Rückfluß. Danach engt man das Reaktionsgemisch ein und kristallisiert den Rückstand aus Glykolmonomethylether um. Man erhält 1,86 g (60 %) der Titelverbindung mit dem Schmelzpunkt 219-221 °C.

**Ansprüche**

1. Verfahren zur Herstellung von Chinoloncarbonsäurederivaten der Formel (I)

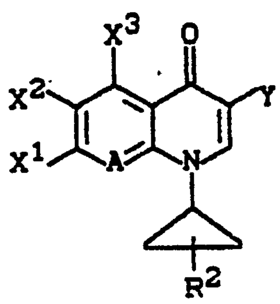

(I)

in der

$R^2$ für Wasserstoff, $C_1$-$C_3$-Alkyl oder Halogen steht,

X' Wasserstoff, $C_1$-$C_3$-Alkyl, Halogen oder $NR^8R^9$ bedeutet, wobei

$R^8$ und $R^9$ gemeinsam mit dem Stickstoffatom, an das sie gebunden sind, einen 5-oder 6-gliedrigen Ring bilden, der als Ringglieder zusätzlich die Atome bzw. die Gruppen

$>NR^{10}$ oder O enthalten kann und der gegebenenfalls ein- bis zweifach

durch $C_1$-$C_3$-Alkyl, Hydroxy, Methoxy oder $NR^{11}R^{12}$ substituiert sein kann, wobei

$R^{10}$ für Wasserstoff, $C_1$-$C_3$-Alkyl, $C_1$-$C_3$-Acyl, insbesondere Acetyl oder t-Butoxycarbonyl steht,
$R^{11}$ für Wasserstoff oder $C_1$-$C_3$-Alkyl steht,
$R^{12}$ Wasserstoff, Acetyl, $C_1$-$C_3$-Alkyl oder t-Butoxycarbonyl bedeutet,
$X^2$ und $X^3$ gleich oder verschieden sein können und für Wasserstoff, Nitro oder Halogen stehen,
A für Stickstoff oder einen Rest C-$X^4$ steht, wobei
$X^4$ für Wasserstoff, $C_1$-$C_3$-Alkyl, Alkoxy mit 1 bis 3 Kohlenstoffatomen, Alkylmercapto mit 1 bis 3 Kohlenstoffatomen, Halogen, Nitro, Cyano oder Alkoxycarbonyl mit bis zu 3 Kohlenstoffatomen im Alkoholteil steht und
Y eine Nitrilgruppe, eine Estergruppe $COOR^5$ oder eine Säureamidgruppe $CONR^6R^7$ darstellt, wobei $R^5$ für $C_1$-$C_3$-Alkyl steht und $R^6$ und $R^7$ gleich oder verschieden sein können und für Wasserstoff oder $C_1$-$C^3$-Alkyl stehen und $R^6$ gegebenenfalls substituiertes Phenyl sein kann,
dadurch gekennzeichnet, daß man in einem ersten Schritt Chinoloncarbonsäurederivate der Formel (II)

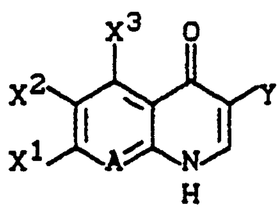

(II)

in der
$X^1$, $X^2$, $X^3$, A und Y die oben angegebene Bedeutung haben
mit Cyclopropanderivaten der Formel (III)

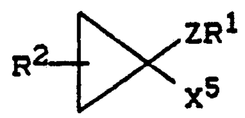

(III)

in der
$X^5$ für Halogen steht,
$R^1$ $C_1$-$C_3$-Alkyl bedeutet und
$R^2$ die oben angegebene Bedeutung hat und
Z für Sauerstoff oder Schwefel steht,
unter $HX^5$-Abspaltung zu 1-Cyclopropylchinoloncarbonsäurederivaten der Formel (IV) nach folgendem Schema umsetzt

II + III

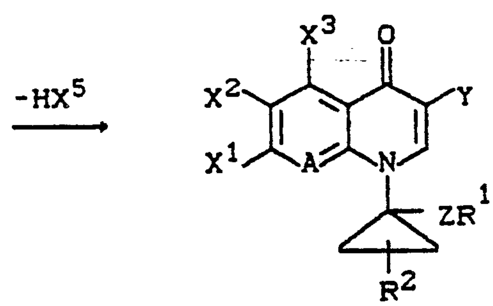


IV,

in der
$X^1$, $X^2$, $X^3$, A, $R^2$, $R^1$ und Y die oben angegebene Bedeutung haben,
die daraufhin durch reduktive Entfernung des Restes $ZR^1$ in die 1-Cyclopropylchinoloncarbonsäurederivate der Formel (I) überführt werden.

2. Verfahren nach Anspruch 1, dadurch gekennzeichnet, daß man die Reaktion der Verbindungen der Formel II mit den Verbindungen der Formel III bei Temperaturen von 20 bis 120 °C, gegebenenfalls in einem Verdünnungsmittel durchführt.

3. Verfahren nach Anspruch 1 und 2, dadurch gekennzeichnet, daß man die Reaktion von Verbindung II mit Verbindung III bei Drucken von 1 bis 100 bar durchführt.

4. Verfahren nach Ansprüchen 1 bis 3, dadurch gekennzeichnet, daß man die Reduktion der Verbindung IV im Falle Z = O mit Natriumcyanoborhydrid, Natriumborhydrid oder Lithiumaluminiumhydrid durchführt.

5. Verfahren nach Anspruch 4, dadurch gekennzeichnet, daß man die Reduktion mit einem 1:1-Gemisch aus Natriumborhydrid und Bortrifluorid-Diethylether-Komplex durchführt.

6. Verfahren nach den Ansprüchen 1 bis 3, dadurch gekennzeichnet, daß man für die Reduktion der Verbindung IV im Fall Z = S Raney-Nickel verwendet.

7. Verbindungen der Formel IV

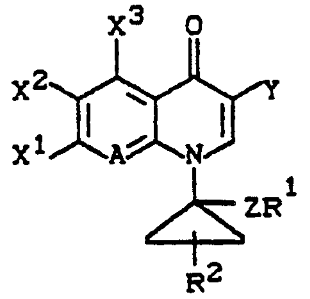

IV,

in der
$X^1$, $X^2$, $X^3$, A, $R^1$, $R^2$, Z und Y die in Anspruch 1 angegebene Bedeutung haben.

8. Verwendung von Verbindungen der Formel IV zur Herstellung von Arzneimitteln.